# EUROPEAN PATENT APPLICATION

(11) **EP 4 349 362 A1**
(43) Date of publication of application: **10.04.2024**
(21) Application number: 22811340.3
(22) Date of filing: 25.05.2022
(51) Int. Cl.: A61K 38/05, A61K 39/395, A61P 35/00, A61P 43/00

(54) **COMBINED USE OF UBENIMEX AND IMMUNE CHECKPOINT INHIBITORS**

(30) Priority: 28.05.2021 JP 2021089984
(71) Applicant: Nippon Kayaku Kabushiki Kaisha, Tokyo 100-0005 (JP)
(72) Inventor: IGO Naoko, Tokyo 115-8588 (JP); NAGAI Daichi, Tokyo 115-8588 (JP); SEKINE Keiko, Tokyo 100-0005 (JP); AIJIMA Michiko, Tokyo 115-8588 (JP)
(74) Representative: Hentrich Patent- & Rechtsanwaltspartnerschaft mbB
(86) International application number: PCT/JP2022/021320
(87) International publication number: WO 2022/250070

(57) **Abstract**

An object of the present invention is to provide a more effective therapeutic method in cancer therapy using the tumor immune mechanism. An object of the present invention is to provide a drug that enhances the production of antitumor, inflammatory, and anti-inflammatory cytokines as the more effective tumor immune therapy. Alternatively, an object of the present invention is to provide a drug that increases tumor-infiltrating lymphocytes as the more effective tumor immune therapy. An antitumor agent to be administered in combination with an anti-PD-1 antibody, an anti-PD-L1 antibody, and/or an anti-CTLA-4 antibody, which are immune checkpoint inhibitors (ICIs), comprising: ubenimex as an active ingredient. The combination therapy of ubenimex and an ICI improves the tumor immune function as compared with the use of each of these alone, and brings many benefits to the treatment of malignant tumors.

## Description

### Technical Field

The present invention relates to a medicament for the treatment of a malignant tumor including administering ubenimex in combination with an anti-PD-1 antibody, an anti-PD-L1 antibody, and/or an anti-CTLA-4 antibody, which are immune checkpoint inhibitors (ICIs).

### Background Art

The relationships among the exacerbation of cancer and inflammatory cells, and immune cells have been clarified in recent years. Therapeutic agents based on the tumor immunity mechanism have been developed on the basis of this knowledge.

Ubenimex ((2S)-2-[(2S,3R)-3-amino-2-hydroxy-4-phenylbutanoylamino]-4-methylpentanoic acid/Bestatin^{®}) is a drug that nonspecifically activates macrophages, T cells, NK cells, and the like, and/or binds to the surfaces of immunocompetent cells through aminopeptidases for activating antitumor immunocompetence to exhibit antitumor action. Ubenimex is clinically used in "the prolongation of life spans by combination with a chemotherapeutic agent for maintenance and reinforcement after the induction of complete remission of adult acute non-lymphocytic leukemia" (Non Patent Literature 1).

Meanwhile, various immune checkpoint molecules that prevent immune response to cancer have been found to be present in cancer cells, and therapeutic agents that remove this immunosuppressive mechanism to obtain antitumor effects have been positively investigated. As immune checkpoint molecules, programmed cell death 1 (PD-1) and programmed cell death ligand 1 (PD-L1), or cytotoxic T-lymphocyte antigen 4 (CTLA-4), and the like are known, and ICIs that target these have been developed. PD-1 is expressed on activated T cells. When PD-1 is bound to PD-L1 or PD-L2, the activity of the T cells is suppressed, the T cells become dysfunctional, and the antitumor immune response is suppressed. PD-L1, which is expressed in cancer cells and the like, binds to PD-1 on activated T cells, and controls the activity of the T cells to suppress antitumor immune response. CTLA-4 is expressed in regulatory T cells (Tregs), activated T cells, and the like, and inhibits the bond between CD80/CD86 on antigen-presenting cells and CD28 on T cells, followed by suppressing the activity of the T cells to suppress antitumor immune response. The main action of immune checkpoint molecules is to suppressively control the activity of T cells. Although the targets of the ICIs are PD-1, PD-L1, and CTLA-4, which are different, the essence of the action thereof is to remove T cell activity suppression. The signal transduction pathways of PD-1/PD-L1 and CTLA-4 are thus important regulatory factors of tumor immune response, and an anti-PD-1 antibody, an anti-PD-L1 antibody, and an anti-CTLA-4 antibody have been developed as a PD-1 inhibitor, a PD-L1 inhibitor, and a CTLA-4 inhibitor, which block these, and achieved remarkable antitumor effects in multiple malignant tumors. As the anti-PD-1 antibody, nivolumab (Opdivo^{®}) and pembrolizumab (Keytruda^{®}) are clinically provided. As the anti-PD-L1 antibody, atezolizumab (Tecentriq^{®}), durvalumab (Imfinzi^{®}), and avelumab (Bavencio^{®}) are clinically provided. As the anti-CTLA-4 antibody, ipilimumab (Yervoy^{®}) is clinically provided.

Since the effects of ICIs are limited in the treatment of malignant tumors, combination therapies, in which multiple antitumor agents are combined, have been attempted to pursue more effective treatment, and the combination therapies including the combinations of antitumor agents such as various cytotoxic drugs and molecular target drugs, the dose, the administration schedules, and the like thereof have been examined. The combined use with drugs related to tumor immunity has also been examined. In Patent Literature 1, for example, the combination therapy of an anti-PD-1 antibody and an anti-CTLA-4 antibody is described. With respect to the combined use of a low molecular drug related to tumor immunity, the combined use of Smac (proapoptotic protein)-like compounds (Smac mimetic compounds (SMCs)), which are apoptosis regulatory factors, and an ICI is described in Patent Literature 2 and Patent Literature 3. In Patent Literature 4, the combined use of an apoptosis protein inhibitor (IAP) antagonist and an anti-PD-1 molecule is described.

### Document List

### Non Patent Literature

Non Patent Literature 1: Pharmaceutical Interview Form "Antineoplastic Drugs: Bestatin Capsules 10 mg and Bestatin Capsules 30 mg", Nippon Kayaku Co., Ltd., October, 2020 (Rev. 7th Edition)

### Patent Literatures

Patent Literature 1: Japanese Patent Application Publication No. 2012-158605
Patent Literature 2: Japanese Patent Application Publication No. 2019-506438
Patent Literature 3: Japanese Patent Application Publication No. 2020-515600
Patent Literature 4: Japanese Patent Application Publication No. 2021-506781

### Summary of Invention

### Technical Problem

An object of the present invention is to provide a more effective therapeutic method in cancer therapy using the tumor immune mechanism. An object of the present invention is to provide a drug that enhances antitumor cytokine production as the more effective tumor immune therapy. Alternatively, an object of the present invention is to provide a drug that increases tumor-infiltrating lymphocytes as the more effective tumor immune therapy.

### Solution to Problem

The present inventors have found that the combination therapy of ubenimex and an ICI improves the tumor immune function as compared with the use of each of these alone and brings many benefits to the treatment of malignant tumors. The present application has the following inventions as the gist.
[1] An antitumor agent to be administered in combination with an anti-PD-1 antibody, an anti-PD-L1 antibody, and/or an anti-CTLA-4 antibody, which are immune checkpoint inhibitors, comprising: ubenimex as an active ingredient.
[2] The antitumor agent according to [1], wherein a dosage of ubenimex is a dosage such that an area under a blood concentration-time curve (AUC) at the time of single administration is 3.00 to 50.00 µg•hr/mL.
[3] The antitumor agent according to [1] or [2], wherein ubenimex is for daily administration for usage.
[4] An antitumor agent to be administered in combination with ubenimex, comprising: an ICI selected from the group consisting of an anti-PD-1 antibody, an anti-PD-L1 antibody, and an anti-CTLA-4 antibody as an active ingredient.
[5] The antitumor agent according to [4], wherein a dosage of ubenimex is a dosage such that an AUC at the time of single administration is 3.00 to 50.00 µg•hr/mL.
   The production of antitumor cytokines, which contribute to the mechanism to activate tumor immunity, are enhanced by the combination of ubenimex and an ICI. The use as an agent for producing antitumor cytokines are therefore provided.
[6] A medicament to be administered in combination with an anti-PD-1 antibody, an anti-PD-L1 antibody, and/or an anti-CTLA-4 antibody, which are immune checkpoint inhibitors, to produce antitumor cytokines, comprising: ubenimex as an active ingredient.
[7] The medicament according to [6], wherein a dosage of ubenimex is a dosage such that an AUC at the time of single administration is 3.00 to 50.00 µg•hr/mL.

The combination of ubenimex and an ICI brings the effect of increasing cytotoxic T cells (CTLs), which are the main factor in the tumor immunity, in a tumor, and contributes to an effective tumor immune therapy. The combination therefore provides the use as an agent for activating tumor-infiltrating lymphocytes.
[8] A medicament to be administered in combination with an anti-PD-1 antibody, an anti-PD-L1 antibody, and/or an anti-CTLA-4 antibody, which are immune checkpoint inhibitors, to increase tumor-infiltrating lymphocytes, comprising: ubenimex as an active ingredient.
[9] The medicament according to [8], wherein a dosage of ubenimex is a dosage such that an AUC at the time of single administration is 3.00 to 50.00 µg•hr/mL.

The combination of ubenimex and an ICI provides a use as a medicament that can be applied to the treatment of extensive malignant tumors.
[10] A medicament comprising ubenimex as an active ingredient, wherein the medicament is administered in combination with an anti-PD-1 antibody, an anti-PD-L1 antibody, and/or an anti-CTLA-4 antibody, which are immune checkpoint inhibitors, to be used for the treatment of a malignant tumor selected from the group consisting of small cell lung cancer, non-small cell lung cancer, esophagus cancer, head and neck cancer, brain tumor, colorectal cancer, colon adenocarcinoma, gastric cancer, breast cancer, hepatocellular cancer, pancreatic cancer, biliary tract cancer, kidney cancer, prostate cancer, bladder cancer, ovarian cancer, cervical cancer, thyroid cancer, and melanoma.
[11] The medicament according to [10], wherein a dosage of ubenimex is a dosage such that an area under a blood concentration-time curve (AUC) at the time of single administration is 3.00 to 50.00 µg•hr/mL.
[12] The medicament according to [10] or [11], wherein ubenimex is for daily administration for usage.

### Effects of Invention

The combination of ubenimex and an ICI brings the reinforcement of the antitumor effect as compared with when each thereof is used alone. The combination can bring the effect of increasing antitumor cytokines or tumor-infiltrating lymphocytes, and provide a more effective tumor immune therapy.

### Brief Description of Drawings

[FIG. 1] A figure showing the transition of the average tumor volumes of groups in a test of the antitumor effect of the administration of ubenimex and the anti-mouse PD-1 antibody (J43) on mouse melanoma cells B16-F0.
[FIG. 2] A figure showing the individual values of the relative tumor volume ratios (T/C) of groups to the tumor volume average value of a control group 15 days after the transplantation in a test of the antitumor effect of the administration of ubenimex and the anti-mouse PD-1 antibody (J43) on mouse melanoma cells B16-F0.
[FIG. 3] A figure showing the individual value plots and average values of plasma IFN-γ of groups by the administration of ubenimex and the anti-mouse PD-1 antibody (J43). The error bars show the standard deviations (*: P < 0.05).
[FIG. 4] A figure showing the individual value plots and the average values of plasma TNF-α of groups by the administration of ubenimex and the anti-mouse PD-1 antibody (J43). The error bars show the standard deviations (*: P < 0.05).
[FIG. 5] A figure showing the individual value plots and the average values of the numbers of CD8-positive T cells per 10000 viable cells dispersed from tumor pieces in single cell suspensions by the administration of ubenimex and the anti-mouse PD-1 antibody (J43). The error bars show the standard deviations.

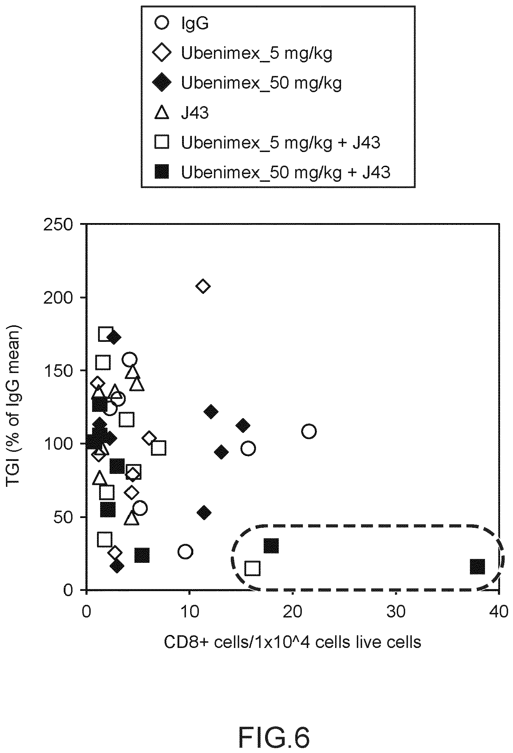

[FIG. 6] A figure showing the correlations between the numbers of CD8-positive T cells and the relative tumor volumes by the administration of ubenimex and the anti-mouse PD-1 antibody (J43). The number of CD8-positive T cells per 10000 viable cells is shown along the horizontal axis, and the relative tumor volume ratio (TGI) of each individual to the tumor volume average of a control group is shown along the vertical axis. The region surrounded with the dotted line shows individuals in which the numbers of CD8-positive T cells are high values in a population that exhibits remarkable tumor growth inhibition.
[FIG. 7] A figure showing the individual value plots of the numbers of regulatory T lymphocytes (Tregs) in peripheral blood by the administration of ubenimex and the anti-mouse PD-1 antibody (J43). The error bars show the standard deviations.
[FIG. 8] A figure showing the correlation between the number of Tregs in peripheral blood and the relative tumor volume by the administration of ubenimex and the anti-mouse PD-1 antibody (J43). The number of Tregs in peripheral blood is shown along the horizontal axis, and the relative tumor volume ratio (TGI) of each individual to the average value of the tumor volume of the control group is shown along the vertical axis. The region surrounded with the dotted line shows individuals in which the numbers of Tregs are low values in a population exhibiting remarkable tumor growth inhibition.
[FIG. 9] A figure showing the transition of the average tumor volumes of groups in a test of the antitumor effect of the administration of ubenimex and the anti-mouse PD-1 antibody (J43) on the mouse colon adenocarcinoma cell line MC38 (*: P < 0.05, **: P < 0.01).
[FIG. 10] A figure showing the transition of the average values of plasma IFN-γ of groups by the administration of ubenimex and the anti-mouse PD-1 antibody (J43) (**: P < 0.01).
[FIG. 11] A figure showing the transition of the average values of plasma TNF-α of groups by the administration of ubenimex and the anti-mouse PD-1 antibody (J43) (*: P < 0.05, **: P < 0.01).
[FIG. 12] A figure showing the transition of the average values of plasma IL-6 of groups by the administration of ubenimex and the anti-mouse PD-1 antibody (J43) (*: P < 0.05).
[FIG. 13] A figure showing the transition of the average values of plasma KC/GRO (IL-8) of groups by the administration of ubenimex and the anti-mouse PD-1 antibody (J43).
[FIG. 14] A figure showing the transition of the average values of plasma IL-10 of groups by the administration of ubenimex and the anti-mouse PD-1 antibody (J43) (**: P < 0.01).

### Description of Embodiments

The gist of the present invention is an antitumor agent in which ubenimex and an ICI are combined. Hereinafter, details thereof will be described.

Ubenimex is (2S)-2-[(2S,3R)-3-amino-2-hydroxy-4-phenylbutanoylamino]-4-methylpentanoic acid, and is a dipeptide compound having aminopeptidase inhibitory activity. In the present invention, ubenimex may be a salt-free body, or may be used as a pharmaceutically acceptable salt such as a hydrochloride or a phosphate. Then, 10 mg of the preparation of ubenimex and 30 mg of the preparation of ubenimex are clinically provided as "Bestatin^{®} capsules", and these may be used.

With respect to the dosage of ubenimex, ubenimex only has to be used in a pharmaceutically effective dose. Ubenimex is preferably applied in a dosage in a range such that the AUC at the time of single administration is more than 3.00 µg•hr/mL and less than 50.00 µg•hr/mL. Ubenimex is preferably applied in a dosage such that the maximum blood concentration (Cmax) is more than 2.0 µg/mL. The dosage is more preferably a dosage such that the AUC is in the range of more than 5.00 µg•hr/mL and less than 50.00 µg•hr/mL, and a dosage such that the AUC is in the range of more than 10.00 µg•hr/mL and less than 50.00 µg•hr/mL is particularly preferable.

Ubenimex may be orally taken, or may be used in a parenteral route such as intravenous administration, for usage. Oral administration is preferable. It is preferable to use ubenimex by daily administration, and it is preferable to orally use ubenimex by daily administration over a therapeutic cycle period.

An anti-PD-1 antibody, an anti-PD-L1 antibody, and an anti-CTLA-4 antibody are therapeutic agents that bind to PD-1, PD-L1, or CTLA-4, which are immune checkpoint molecules for regulating tumor immunity, block the PD-1/PD-L1 or CTLA-4 signal transduction pathway, and remove the mechanism to suppress activated T cells to obtain antitumor effect. As long as the anti-PD-1 antibody, the anti-PD-L1 antibody, or the anti-CTLA-4 antibody in the present invention is an antibody that binds to PD-1, PD-L1 or CTLA-4 to have the removal of the function of suppressing tumor immunity, the anti-PD-1 antibody, the anti-PD-L1 antibody, or the anti-CTLA-4 antibody can be applied without particular limitation.

Many antitumor agents are marketed or being developed as the anti-PD-1 antibody, the anti-PD-L1 antibody, and the anti-CTLA-4 antibody, and these may be applied.

Examples of the anti-PD-1 antibody include nivolumab (Opdivo^{®}) and pembrolizumab (Keytruda^{®}). Pidilizumab (CT-011), PDR-001, JS001, STI-A1110, AMP-224, AMP-514 (MEDI0680), and the like may be used.

Examples of the anti-PD-L1 antibody includes atezolizumab (Tecentriq^{®}), durvalumab (Imfinzi^{®}), and avelumab (Bavencio^{®}). BMS-936559 (MDX1105), LY3300054, and the like may be used.

Examples of the anti-CTLA-4 antibody include ipilimumab (Yervoy^{®}). Tremelimumab (CP-675206) or the like may be used.

In the present invention, it is preferable that the ICI be used in combination with ubenimex be the anti-PD-1 antibody and/or the anti-PD-L1. The combined use of ubenimex and the anti-PD-1 antibody is more preferable.

The anti-PD-1 antibody, the anti-PD-L1 antibody, or the anti-CTLA-4 antibody only has to be used in a pharmaceutically effective dose. For example, if nivolumab (Opdivo^{®}), which is an anti-PD-1 antibody, is used, 240 mg per once is usually applied by intravenous drip at intervals of two weeks. If pembrolizumab (Keytruda^{®}) is used, 200 mg per once is usually applied by intravenous drip at intervals of three weeks. When atezolizumab (Tecentriq^{®}), which is an anti-PD-L1 antibody, is used, 840 mg per once is usually applied by an intravenous drip at intervals of two weeks, or 1200 mg per once is usually applied by intravenous drip at intervals of three weeks. If durvalumab (Imfinzi^{®}) is used, 10 mg/kg per once is usually applied by intravenous drip at intervals of two weeks, or 1500 mg per once is usually applied by intravenous drip at intervals of three weeks. If avelumab (Bavencio^{®}) is used, 10 mg/kg per once is applied by intravenous drip at intervals of two weeks. If ipilimumab (Yervoy^{®}), which is an anti-CTLA-4 antibody, is used, 3 mg/kg per once is usually applied by intravenous drip four times at intervals of three weeks.

The present invention relates to combination therapy in which ubenimex and the ICI are used in combination. The combination therapy according to the present invention means that ubenimex and the ICI are administered simultaneously or sequentially, and includes being a series of therapeutic methods comprising the administration of ubenimex and the ICI in a therapeutic regimen including the administration of multiple drugs. The simultaneous administration includes the administration of both drugs at substantially the same time. The sequential administration includes administering one drug over one day or several days, and then administering the other drug after an optional washout period. In the present invention, combined usage is preferred in which ubenimex is daily administered and the ICI is administered every 1 to 3 weeks during the administration period thereof.

The combination therapy of ubenimex and the ICI of the present invention is provided for treating a malignant tumor. Examples of the malignant tumor include small cell lung cancer, non-small cell lung cancer, esophagus cancer, head and neck cancer, brain tumor, colorectal cancer, colon adenocarcinoma, gastric cancer, breast cancer, hepatocellular cancer, pancreatic cancer, biliary tract cancer, kidney cancer, prostate cancer, bladder cancer, ovarian cancer, cervical cancer, thyroid cancer, and melanoma. For example, it is preferable that the combination therapy be used for treating non-small cell lung cancer.

The combination therapy of ubenimex and the ICI brings a higher effect of inhibiting tumor growth than the antitumor effect of the administration of each thereof alone. That is, the present invention provides a new use of ubenimex in which ubenimex is used in combination with the ICI. Alternatively, the present invention includes a new use of the ICI in which the ICI is used in combination with ubenimex.

Ubenimex and the ICI are drugs that achieves tumor growth inhibition through antitumor immunity. Ubenimex is a drug that brings the activation of macrophages, NK cells, T cells, bone marrow cells, and the like, and serially activates the immune system network by direct antitumor effect of these or with various cytokines such as interleukins 1 and 2 produced from here to exhibit antitumor effect. The ICI is a drug that inhibits PD-1/PD-L1 and/or CTLA-4 from binding, and removes immune suppression signals to obtain an antitumor effect by activating T cells that is specific to a cancer antigen and activating the cytotoxicity.

The present invention has found that the combined administration of ubenimex and the ICI reinforces the antitumor immunity as compared with the use of each single agent, and also includes providing a new use thereof as an antitumor immunopotentiator.

As another aspect of the present invention, the combined administration of ubenimex and the ICI has the effect of reinforcing the production of antitumor cytokines such as interferon (INF)-γ or TNF-α, inflammatory cytokines such as IL-6 or IL-8, and anti-inflammatory cytokines such as IL-10. As described above, it is known that ubenimex activates immune cells, and brings the production of cytokines such as IL-1 and IL-2. The administration of ubenimex and the ICI in combination, however, enhances the production of antitumor and inflammatory cytokines and the like clearly as compared with the administration of ubenimex alone. Accordingly, the use as an agent for reinforcing the production of antitumor and inflammatory cytokines and the like containing ubenimex as an active ingredient by administering the agent in combination with the ICI is provided.

The dosage of ubenimex only has to be used in a pharmaceutically effective dose for inducing the production of antitumor and inflammatory cytokines and the like. Ubenimex is preferably applied in a dosage such that the AUC at the time of single administration is in the range of more than 3.00 µg•hr/mL and less than 50.00 µg•hr/mL. Ubenimex is preferably applied in a dosage such that the Cmax is more than 2.0 µg/mL. The dosage is more preferably a dosage such that the AUC is in the range of more than 5.00 µg•hr/mL and less than 50.00 µg•hr/mL, and the dosage in the range of more than 10.00 µg•hr/mL and less than 50.00 µg•hr/mL is particularly preferable.

As another aspect of the present invention, the combined administration of ubenimex and the ICI has the effect of improving the induction of tumor-infiltrating lymphocytes. As described above, ubenimex and the ICI have the action of inducing and activating CTLs. The administration of ubenimex and the ICI in combination, however, brings the induction of more tumor-infiltrating lymphocytes as compared with the administration of ubenimex alone. The use as an agent for reinforcing the induction of the tumor-infiltrating lymphocytes containing ubenimex as an active ingredient by administering the agent in combination with the ICI is therefore provided.

With respect to the dosage of ubenimex, ubenimex only has to be used in a pharmaceutically effective dose for the induction of tumor-infiltrating lymphocytes. Ubenimex is preferably applied in a dosage in the range such that the AUC at the time of single administration is more than 3.00 µg•hr/mL and less than 50.00 µg•hr/mL. Ubenimex is preferably applied in a dosage such that the Cmax is more than 2.0 µg/mL. The dosage is more preferably a dosage in the range such that the AUC is more than 5.00 µg•hr/mL and less than 50.00 µg•hr/mL, and a dosage in the range of more than 10.00 µg•hr/mL and less than 50.00 µg•hr/mL is particularly preferable.

As another aspect of the present invention, the combined administration of ubenimex and the ICI has the effect of suppressing the induction of Tregs, which are a suppressor in the antitumor immunity. It is effective to make the tumor growth inhibition through the antitumor immunity function with the induction and activation of CTLs, which attack cancer cells directly, simultaneously with the removal of the tumor immunity suppression mechanism. As described above, the combined administration of ubenimex and the ICI according to the present invention can suppress the induction of Tregs with a mechanism to guide tumor-infiltrating lymphocytes to cancer cells, and provide an efficient antitumor immune therapy.

With respect to the dosage of ubenimex, ubenimex only has to be used in a pharmaceutically effective dose for suppressing the induction of Tregs. Ubenimex is preferably applied in a dosage in the range such that the AUC at the time of single administration is more than 3.00 µg•hr/mL and less than 50.00 µg•hr/mL. Ubenimex is preferably applied in a dosage such that the Cmax is more than 2.0 µg/mL. The dosage is more preferably a dosage in the range such that the AUC is more than 5.00 µg•hr/mL and less than 50.00 µg•hr/mL, and a dosage in the range of more than 10.00 µg•hr/mL and less than 50.00 µg•hr/mL is particularly preferable.

The combination therapy of ubenimex and the ICI of the present invention may be the combination therapy in which an additional antitumor agent may be further combined. The additional antitumor agent is not particularly limited, and a pharmaceutical approved as an antitumor agent can be used. For example, alkylating agents such as cyclophosphamide, ifosfamide, and temozolomide. Anthracycline-based antitumor agents such as doxorubicin, epirubicin, pirarubicin, amrubicin, and a doxorubicin-encapsulating liposome preparation (Doxil^{®}). Antitumor platinum complexes such as cisplatin, carboplatin, and oxaliplatin. Etoposides such as etoposide, etoposide phosphate, and teniposide. Camptothecins such as irinotecan and nogitecan. Taxanes such as paclitaxel, docetaxel, cabazitaxel, and albumin-suspended paclitaxel (Abraxane ^{®}). Vinca alkaloids such as vincristine, vinblastine, vindesine, and vinorelbine. Tubulin polymerization inhibitors such as eribulin. Nucleic acid antimetabolites such as 5-fluorouracil, tegafur, a tegafur-uracil combination (UFT), a tegafur-gimeracil-oteracil potassium combination (S-1), a trifluridine-tipiracil combination (Lonsurf^{®}), furtulon, capecitabine, gemcitabine, cytosine arabinoside, and azacitidine. Folic acid antimetabolites such as methotrexate and pemetrexed. Steroids such as prednisolone and dexamethasone.

Tyrosine kinase inhibitors such as erlotinib, lapatinib, gefitinib, afatinib, osimertinib, axitinib, sunitinib, sorafenib, regorafenib, lenvatinib, alectinib, crizotinib, and pazopanib. Proteasomal inhibitors such as bortezomib, carfilzomib, and ixazomib. Cyclin-dependent kinase inhibitors such as palbociclib. mTOR inhibitors such as everolimus and temsirolimus. Anti-growth factors (receptor) antibodies such as trastuzumab, cetuximab, necitumumab, bevacizumab, panitumumab, and ramucirumab. Other known antitumor agents may be used.

It is preferable that the additional antitumor agent be used in combination by known usage in a pharmaceutically effective dose.

If the present invention is applied as a therapeutic agent for non-small cell lung cancer, it is preferable to apply the present invention in combination with a known therapeutic method. For example, it is preferable to apply the present invention in combination with paclitaxel, albumin-suspended paclitaxel (Abraxane^{®}), docetaxel, a tegafur-gimeracil-oteracil potassium combination (S-1), gemcitabine, pemetrexed, vinorelbine, gefitinib, erlotinib, afatinib, osimertinib, necitumumab, and the like as a concomitant drug. It is also preferable to further combine chemotherapy in which an antitumor platinum complex that is cisplatin or carboplatin is combined with these antitumor agents. The present invention is more preferably the combined use of paclitaxel and an antitumor platinum complex, the combined use of albumin-suspended paclitaxel (Abraxane^{®}) and an antitumor platinum complex, the combined use of pemetrexed and an antitumor platinum complex, or the combined use of a tegafur-gimeracil-oteracil potassium combination (S-1) and an antitumor platinum complex. It is preferable for these additional antitumor agents to be used in combination to be applied in usage and a dosage the efficacy of which is confirmed in non-small cell lung cancer.

### Examples

Hereinafter, the present invention will be described in further detail with the Examples. Materials used in the Examples were acquired by the following and provided to tests.

### (1) Tumor cells

Mouse melanoma cell line B16-F0 was obtained from the ATCC (the American Type Culture Collection). The cells obtained by culturing this in DMEM (Thermo Fisher Scientific K.K.) containing 10% fetal calf serum (Corning Incorporated) and 100 µg/mL kanamycin sulfate in a CO₂ incubator (37°C, in 5% carbon dioxide) were used.

The mouse colon adenocarcinoma cell line MC38 was obtained from genOway. The cells obtained by culturing this in DMEM (Thermo Fisher Scientific K.K.) containing 10% fetal calf serum (Corning Incorporated), 2 mM L-glutamine (Thermo Fisher Scientific K.K.), 10 mM HEPES (Thermo Fisher Scientific K.K.), 1 mM sodium pyruvate (FUJIFILM Wako Pure Chemical Corporation), 0.1 mM MEM Non-Essential Amino Acids Solution (Thermo Fisher Scientific K.K.), and penicillin/streptomycin (FUJIFILM Wako Pure Chemical Corporation) in a CO2 incubator (37°C, in 5% carbon dioxide) were used.

### (2) Test substances

Ubenimex was obtained from Nippon Kayaku Co., Ltd.
An anti-mouse PD-1 antibody (clone J43) and an isotype control antibody IgG (polyclonal Armenian hamster IgG) were obtained from Bio X Cell.
Ubenimex was dissolved in distilled water for injection (Otsuka Pharmaceutical Factory, Inc.) and used. The anti-mouse PD-1 antibody and the IgG were diluted with PBS (-) (Thermo Fisher Scientific K.K.) and used.

### [Example 1]

The antitumor effect of the combined administration of ubenimex and the anti-mouse PD-1 antibody was evaluated with a model in which the mouse melanoma cells B16-F0 were subcutaneously transplanted. Fluctuation in cytokines and immune system markers was confirmed at the same time.

First, 2 × 10⁵ cells of B16-F0 were subcutaneously transplanted to female C57BL/6N mice (Charles River Laboratories Japan, Inc.) to produce a subcutaneous transplantation model.

Ubenimex was orally administered to subcutaneous transplantation model B16-F0 mice once a day, 14 times in total, in a dosage of 5 or 50 mg/kg from the day following the transplantation to 14 days after transplantation. Furthermore, an anti-mouse PD-1 antibody was intraperitoneally administered in a dosage of 200 µg/body 4, 7, 11, and 14 days after the transplantation. An IgG antibody, which was an isotype control of the anti-mouse PD-1 antibody, was administered as a control under the same conditions as the anti-mouse PD-1 antibody. The treated group structure was put into Table 1.

**[Table 1]**

| Group | Test substance | Dose | Administration schedule | The number of animals |
|---|---|---|---|---|
| 1 | Control (Armenian hamster IgG) | 200 µg / body | Twice weekly* | 8 |
| 2 | Ubenimex | 5 mg / kg | Once daily | 8 |
| 3 | Ubenimex | 50 mg / kg | Once daily | 8 |
| 4 | Anti-PD-1 antibody (J43) | 200 µg / body | Twice weekly* | 8 |
| 5 | Ubenimex | 5 mg / kg | Once daily | 8 |
| | Anti-PD-1 antibody (J43) | 200 µg / body | Twice weekly* | |
| 6 | Ubenimex | 50 mg / kg | Once daily | 8 |
| | Anti-PD-1 antibody (J43) | 200 µg / body | Twice weekly* | |

| | | | | |
|---|---|---|---|---|
| *: 4, 7, 11, and 14 days after transplantation | | | | |

The major axis and the minor axis of the tumors were measured with a digital micrometer caliper (Mitutoyo Corporation), and the tumor volumes (mm³) were calculated by the expression "major axis × minor axis²/2". The relative tumor volume (T/C) of each treated group to the control group was calculated by the expression "average tumor volume of the test substance-treated group/average tumor volume of control group × 100" using the tumor volume value 15 days after the transplantation.

The animals in all the cases were subjected to autopsies 15 days after the transplantation, namely on the day following the final administration. Blood was collected from the jugular vein with syringes to which EDTA-2K solution was added and injection needles on the autopsy day, and the total white blood cell count was measured in the multiple automatic blood cell analyzer XN-2000V (SYSMEX CORPORATION). Blood was collected from the abdominal aorta with syringes to which heparin sodium solution was added and injection needles under anesthesia, and the tumors were successively collected. The blood and the tumor tissues were used for immunophenotyping. The plasma obtained by centrifuging the blood was measured for cytokines by ELISA.

The control group and the treated groups, and the combined use groups and the single agent groups, were subjected to the Wilcoxon tests using the statistical analysis software EXSUS (ver. 10.0, EPS Corporation) for statistical analysis. Two-sided statistical analysis was performed at a significance level of P < 0.05.

### [Example 1.1] Antitumor effect

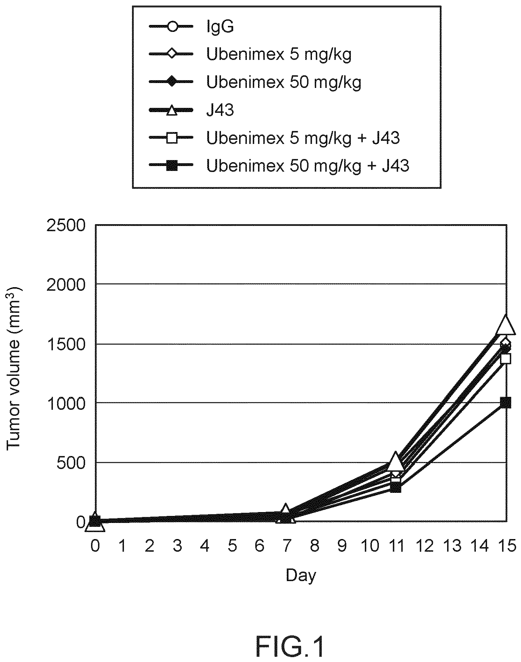

The tumor growth transition in the treated groups was shown in FIG. 1. In the relative average tumor volume (T/C) to the control group (IgG was administered) 15 days after the transplantation, the treated groups of 5 mg/kg and 50 mg/kg of ubenimex and the treated group of the anti-PD-1 antibody did not exhibit tumor growth inhibition effect over the control group, whereas the T/C of the combined use group of a high dosage of ubenimex and the anti-PD-1 antibody were 68%, and a tumor growth inhibition effect was exhibited. It was confirmed that the combined use of ubenimex and the anti-PD-1 antibody exhibited a tumor growth inhibition effect that was not observed in the administration of each thereof alone.

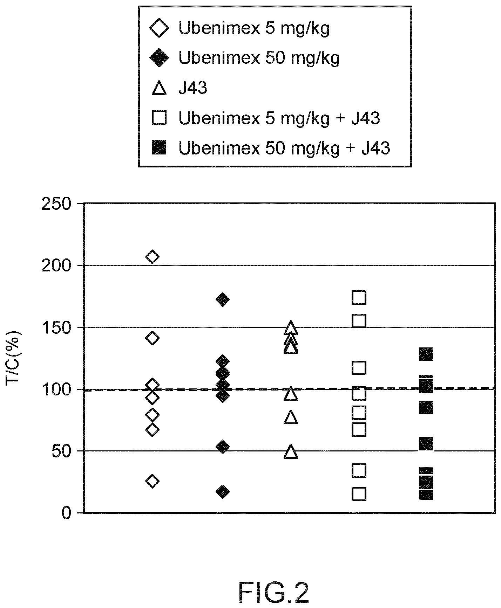

The antitumor effects were analyzed using the individual values of the relative tumor volumes in Example 1.1 to reveal that, in the administration of the single agents, individuals that exhibited a T/C 15 days after the transplantation of less than 100% were half or less of the cases of the group, whereas in the combined use of a low dosage of ubenimex and the anti-PD-1 antibody and the combined use of a high dosage of ubenimex and the anti-PD-1 antibody, individuals that exhibited a T/C 15 days after the transplantation of less than 100% were both five of eight, which was more than half (FIG. 2).

### [Example 1.2] Antitumor cytokine production

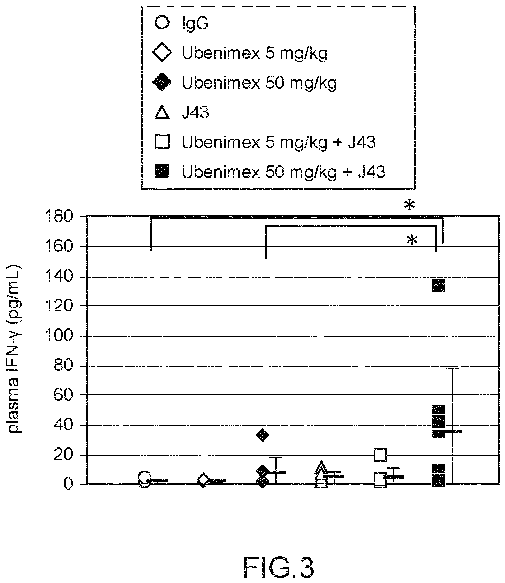

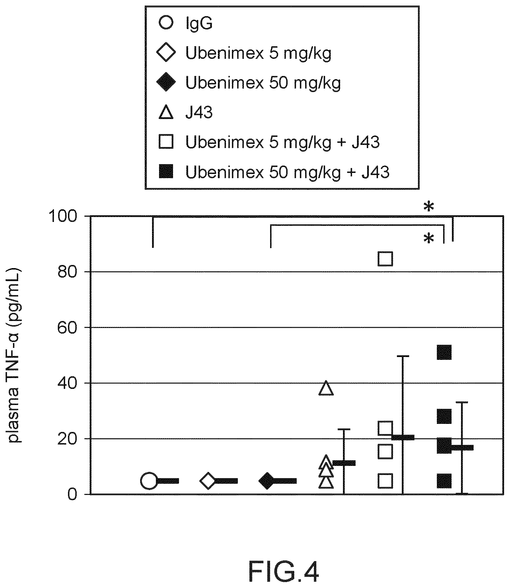

As antitumor cytokines in plasma in treated groups, the concentration of IFN-γ and TNF-α were measured with a LBIS Mouse IFN-γ ELISA kit and a LBIS Mouse TNF-α ELISA kit (FUJIFILM Wako Shibayagi Corporation). The results were shown in FIGs. 3 and 4.

The combined use group of a high dosage of ubenimex and the anti-PD-1 antibody exhibited a clear effect of reinforcing IFN-γ production as compared with the control group, the treated groups of ubenimex alone, and the treated group of the anti-PD-1 antibody alone. The combined use group of a high dosage of ubenimex significantly increased as compared with the control group and the group of a high dosage of ubenimex alone (P value: 0.02, FIG. 3).

The combined use group of ubenimex and the anti-PD-1 antibody exhibited an effect of reinforcing TNF-α production as compared with the control group, the treated groups of ubenimex alone, and the treated group of the anti-PD-1 antibody alone. In the combined use group of a high dosage of ubenimex and the anti-PD-1 antibody, the TNF-α production significantly increased as compared with the control group and the group of a high dosage of ubenimex alone (P value: 0.045, FIG. 4).

IFN-γ and TNF-α are known as antitumor cytokines, which serially activate the immune system network, and contribute to antitumor effect. Since the combined use group of ubenimex and the anti-PD-1 antibody exhibited a tumor growth inhibition effect, it was suggested that IFN-γ and TNF-α may have contributed to tumor growth inhibition.

### [Example 1.3] Immunophenotyping of tumor-infiltrating lymphocytes

After the confirmation of the antitumor effects, the tumor tissues collected by autopsies were analyzed for tumor-infiltrating lymphocytes by flow cytometry.

An anti-mouse CD45 antibody, an anti-mouse CD3 antibody, an anti-mouse CD4 antibody, an anti-mouse CD8 antibody, an anti-mouse/human CD11b antibody, an anti-mouse CD25 antibody, an anti-mouse/human FOXP3 antibody, an anti-mouse CD16/32 antibody (BioLegend, Inc.), a Lysing Buffer (Becton, Dickinson and Company), a True-Nuclear Transcription Factor Buffer Set (BioLegend, Inc.), and a Tumor Dissociation Kit, mouse (Miltenyi Biotec K.K.) were used for immunophenotyping.

Each tumor piece was finely cut in an enzyme solution of the Tumor Dissociation Kit, and the fragments of the tumor piece were mechanically dispersed in a gentleMACS Octo Dissociator and incubated at 37°C for 40 minutes while the fragments were gently shaken. After the incubation, mechanical dispersion treatment was performed again. Then, 10% FBS-containing RPMI1640 was added. The dispersion was passed through a 70-µm cell strainer. After the centrifugation treatment, cell clusters were resuspended in 10% FBS-containing RPMI1640, and this was produced into a single cell suspension subjected to dispersion preparation from the tumor piece. A portion of the single cell suspension was separated and subjected to hemolysis treatment in the Lysing Buffer, and Fc receptors were subjected to blocking treatment with the anti-mouse CD16/32 antibody. Cell surface markers CD45, CD3, CD4, CD8, and CD11b were then stained and measured with the flow cytometer LSRII (Becton, Dickinson and Company).

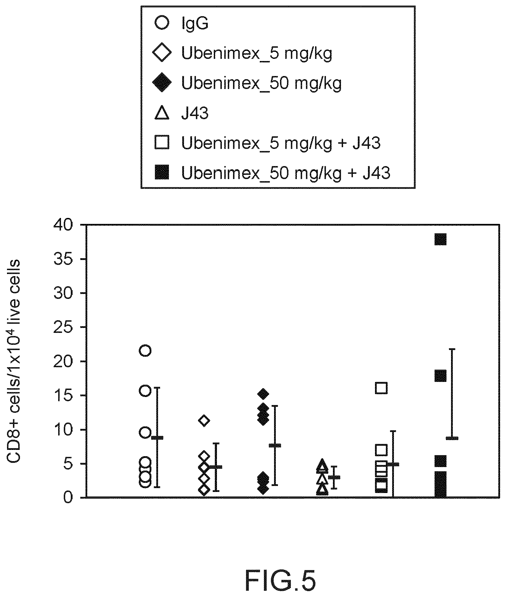

CD8-positive T cells (CD45+, CD3+, and CD8+), which were CTLs, were used as indices for the evaluation. The total number of the CD8-positive T cells among the total number of viable cells in the single cell suspension was calculated using the CD8-positive T cell rate calculated from the count of the total viable cell fraction and the count of CD8-positive T cells in the single cell suspension in the flow cytometer measurement. The CD8-positive T cell rate was calculated as the number of the CD8-positive T cells per 10000 viable cells. The results of the numbers of CD8-positive T cells in the treated groups were shown in FIG. 5.

Individuals that exhibited a large number of CD8-positive T cells in the tumor were observed in the combined use group of a high dosage of ubenimex and the anti-PD-1 antibody. When the correlation between the number of CD8-positive T cells and the relative tumor volume was examined, it was observed that individuals in the combined use groups that exhibited remarkable tumor growth inhibition matched individuals that exhibited large numbers of CD8 positive T cells. The action of inducing CD8-positive T cells, which contributed to tumor growth inhibition effect, was observed not only in the combined use of a high dosage of ubenimex but also in the combined use of a low dosage of ubenimex (FIG. 6). The CD8-positive T cells have action of damaging tumor cells as CTLs. Accordingly, the combined use of ubenimex and the anti-PD-1 antibody can induce CTLs in the tumor and provide tumor growth inhibition effect.

### [Example 1.4] Immunophenotyping of lymphocytes in peripheral blood

The blood collected by the autopsies after the confirmation of the antitumor effects was analyzed for lymphocytes in peripheral blood by flow cytometry.

The peripheral blood was subjected to hemolysis treatment with a Lysing Buffer to prepare peripheral blood mononuclear cells (PBMCs), the Fc receptors were then blocked with the anti-mouse CD16/32 antibody, and the cell surface markers CD45, CD3, CD4, CD8, and CD25 were stained. The PBMCs were fixed and permeabilized, and the nuclear factor FOXP3 was stained, and measurement was performed with the flow cytometer LSRII (Becton, Dickinson and Company).

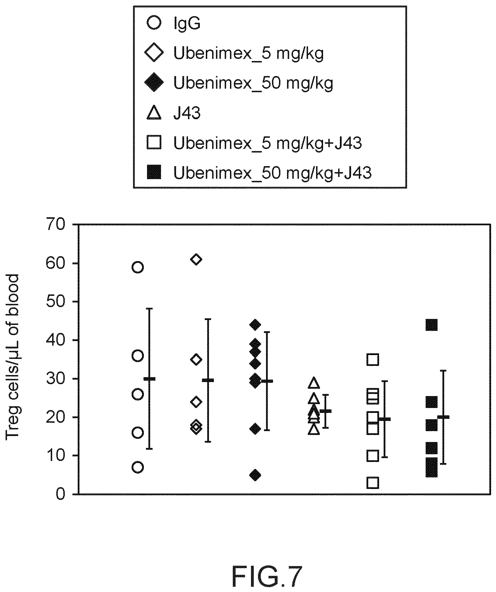

Tregs identified using CD45+, CD3+, CD4+, CD25+, and FOXP3+ were used as indices for evaluation. The existence rate of Tregs in CD45-positive cells was calculated, and the number of Tregs per 1 µL of the peripheral blood was calculated from the value of the total white blood cell count. The results of the numbers of Tregs in the treated groups were shown in FIG. 7.

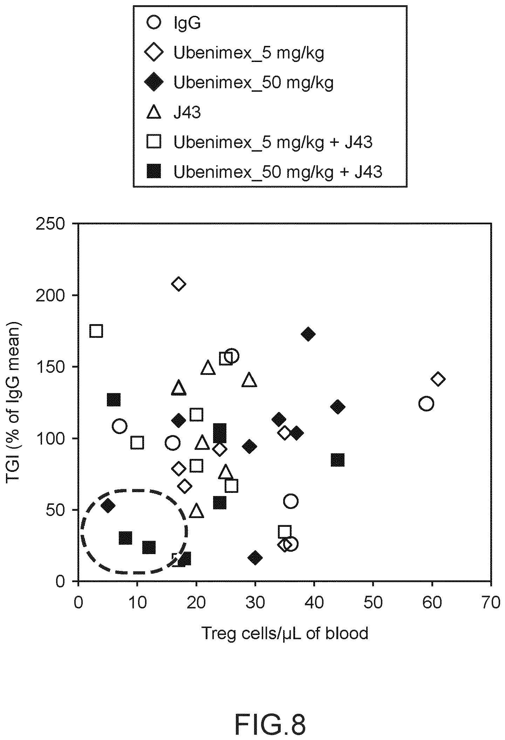

In the treated group of the anti-PD-1 antibody alone and the combined use group of the anti-PD-1 antibody and ubenimex in low and high dosages, the numbers of Tregs in the peripheral blood were low values (FIG. 7). Meanwhile, no change was seen in the treated group of ubenimex alone. When the correlation between the number of Tregs and the relative tumor volume was seen, it was observed that individuals in the combined groups that exhibit remarkable tumor growth inhibition match individuals that exhibit small numbers of Tregs. The action of suppressing the induction of Tregs, which contributes to tumor growth inhibition effect, was observed not only in the combined use of a high dosage of ubenimex but also in the combined use of a low dosage of ubenimex (FIG. 8). Tregs have the action of exhibiting the inhibition of the activation of or cytotoxicity to CTLs, having a main function in the tumor immunity. Accordingly, the action of reducing Tregs is the effect of promoting the tumor immunity and contributing to tumor growth inhibition.

The results of immunophenotyping reveals that in the treated groups of ubenimex and the anti-PD-1 antibody in combination, the induction of Tregs is suppressed, and CTLs in tumors are induced, and it can be expected that the tumor immune action in tumor local area can be promoted.

It was shown from the above that the combined administration of ubenimex and the anti-PD-1 antibody performed the activation of multiple tumor immune system networks such as an increase in the number of CTLs, increase in the production of antitumor cytokines, and the removal of the tumor immune tolerant status based on lymphocytes-activating action that ubenimex had, and was able to exhibit antitumor effect.

### [Example 2] Mouse blood concentration transition

First, 9 nine-week-old fasted female C57BL/6N mice (Charles River Laboratories Japan, Inc.) in each group were subjected to the single oral administration of 5 mg/kg or 50 mg/kg of ubenimex. Blood was collected at 9 time points, 5, 10, and 30 minutes, 1, 3, 6, 8, 12, and 24 hours after the administration. Then, 0.1 mL of blood was collected from the jugular vein under isoflurane anesthesia from 5 minutes to 6 hours after the administration, and the whole blood was collected from the vena cava under isoflurane anesthesia 8 hours or more after the administration. To secure n = 3 at each time point, the blood collection was sparse blood collection in which blood was collected from one individual at 3 time points, and three animals were replaced as one set. Plasma was prepared from the obtained blood, and the plasma ubenimex concentration was measured with a liquid chromatography mass spectrometer (LC/MS/MS). The pharmacokinetic parameters in each dose are shown in Table 2.

**[Table 2]**

| Dose | Tₘₐₓ | Cₘₐₓ | AUC_{inf} | MRT_{inf} |
|---|---|---|---|---|
| mg/kg | hr | µg/mL | µg·h/mL | hr |
| 5 | 0.083 | 4.6 | 3.24 | 0.872 |
| 50 | 0.17 | 44 | 47.7 | 0.918 |
| Ratio of 50 mg/5 mg | - | 9.57 times | 14.7 times | 1.05 times |

The Cmax was directly proportional to the dose, and the MRTinf was constant regardless of the dose. Although the ratio of 50 mg/5 mg in the AUCinf tended to be higher than the dose ratio, the dosage proportionality was generally obtained in a dosage of 5 to 50 mg/kg in the mouse.

### [Example 3] Antitumor effect

The antitumor effect of the combined administration of ubenimex and the anti-mouse PD-1 antibody was evaluated with a subcutaneous transplantation model of the mouse colon adenocarcinoma cells MC38.

Then, 1 × 10⁵ cells of MC38 were subcutaneously transplanted into female C57BL/6N mice (Charles River Laboratories Japan, Inc.) to produce the subcutaneous transplantation model. Ubenimex was orally administered to each subcutaneous transplantation model mouse of MC38 in a dosage of 0.5 or 5 mg/kg once a day, 24 times in total, from the day following the transplantation to 24 days after the transplantation. Furthermore, the anti-mouse PD-1 antibody was intraperitoneally administered in a dosage of 50 µg/body 7,11,14,18,21, and 24 days after the transplantation. An IgG antibody that was an isotype control of the anti-mouse PD-1 antibody was administered as a control under the same conditions as the anti-mouse PD-1 antibody. The treated group structure was put into Table 3.

**[Table 3]**

| Group | Test substance | Dose | Administration schedule | The number of animals |
|---|---|---|---|---|
| 1 | Control (Armenian hamster IgG) | 50 µg / body | Twice weekly* | 7 |
| 2 | Ubenimex | 0.5 mg / kg | Once daily | 9 |
| 3 | Ubenimex | 5 mg / kg | Once daily | 10 |
| 4 | Anti-PD-1 antibody (J43) | 50 µg / body | Twice weekly* | 9 |
| 5 | Ubenimex | 0.5 mg / kg | Once daily | 9 |
| | Anti-PD-1 antibody (J43) | 50 µg / body | Twice weekly* | |
| 6 | Ubenimex | 5 mg / kg | Once daily | 9 |
| | Anti-PD-1 antibody (J43) | 50 µg / body | Twice weekly* | |

| | | | | |
|---|---|---|---|---|
| *: 7, 11, 14, 18, 21, and 24 days after transplantation | | | | |

The major axis and the minor axis of the tumors were measured with a digital micrometer caliper (Mitutoyo Corporation), and the tumor volumes (mm³) were calculated by the expression "major axis × minor axis²/2". The relative tumor volume (T/C) of each treated group to the control group was calculated by the expression "average tumor volume of the test substance-treated group/average tumor volume of control group × 100" using the tumor volume value 25 days after the transplantation.

The control group and the treated groups, and the combined use groups and the single agent groups, were subjected to the Wilcoxon tests using the statistical analysis software EXSUS (ver. 10.0, EPS Corporation) for statistical analysis. Two-sided statistical analysis was performed at significance levels of P < 0.05 and P < 0.01.

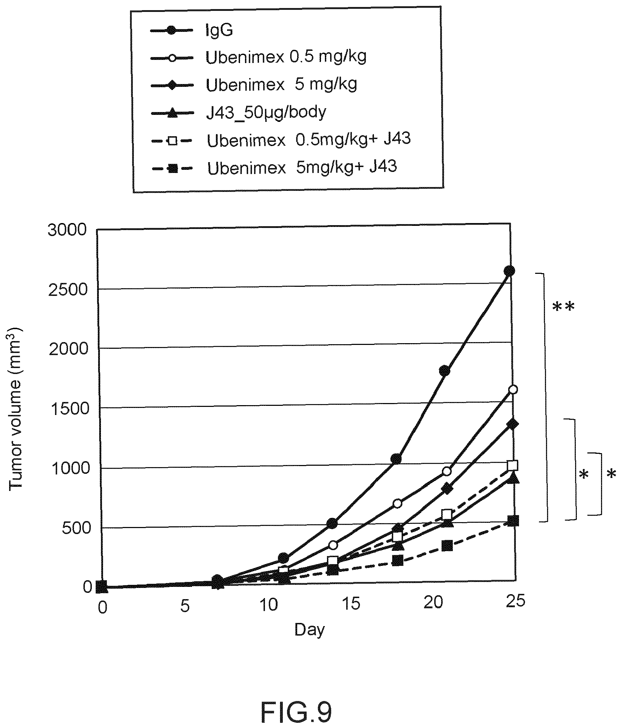

The tumor growth transition in the treated groups was shown in FIG. 9. With respect to the relative average tumor volume of the treated groups of ubenimex alone or the treated group of the anti-PD-1 antibody alone to the control group (IgG was administered) 25 days after the transplantation (T/C), the T/C of the 0.5 mg/kg ubenimex group was 62%, the T/C of the 5 mg/kg ubenimex group was 51%, the T/C of the anti-PD-1 antibody group was 33%, and all thereof exhibited antitumor effect. With respect to the T/C of the combined use groups, the T/C of the combined use group of 0.5 mg/kg of ubenimex and the anti-PD-1 antibody was 37%, and the T/C of the combined use group of 5 mg/kg of ubenimex and the anti-PD-1 antibody was 19%.

The dosage-dependent antitumor effect of ubenimex was confirmed. In the combined use group of 5 mg/kg of ubenimex and the anti-PD-1 antibody, an effect that was stronger than the single agents was observed (the P value to the control group: 0.002, the P value to the 5 mg/kg ubenimex group: 0.015, the P value to the combined use group of 0.5 mg/kg of ubenimex and the anti-PD-1 antibody: 0.039, FIG. 9).

### [Example 4] Cytokine production

The plasma cytokine transition by the combined use of ubenimex and the anti-mouse PD-1 antibody was evaluated using a subcutaneous transplant model of the mouse colon adenocarcinoma cells MC38.

Then, 1 × 10⁵ cells of MC38 were subcutaneously transplanted to female C57BL/6N mice (Charles River Laboratories Japan, Inc.) to produce the subcutaneous transplant model. Ubenimex was orally administered to the MC38 subcutaneous transplant model mouse in a dosage of 5 mg/kg once a day, 24 times in total, from the day following the transplantation to 24 days after the transplantation. Furthermore, the anti-mouse PD-1 antibody was intraperitoneally administered in a dosage of 50 µg/body 7, 11, 14, 18, 21, and 24 days after the transplantation. The IgG antibody, which was an isotype control of the anti-mouse PD-1 antibody, was administered as a control under the same conditions as the anti-mouse PD-1 antibody. The treated group structure was put into Table 4.

**[Table 4]**

| Group | Test substance | Dose | Administration schedule | The number of animals |
|---|---|---|---|---|
| 1 | Control (Armenian hamster IgG) | 50 µg / body | Twice weekly* | 6 |
| 2 | Ubenimex | 5 mg / kg | Once daily | 5 |
| 3 | Anti-PD-1 antibody (J43) | 50 µg / body | Twice weekly* | 6 |
| 4 | Ubenimex | 5 mg / kg | Once daily | 6 |
| | Anti-PD-1 antibody (J43) | 50 µg / body | Twice weekly* | |

| | | | | |
|---|---|---|---|---|
| *: 7, 11, 14, 18, 21, and 24 days after transplantation | | | | |

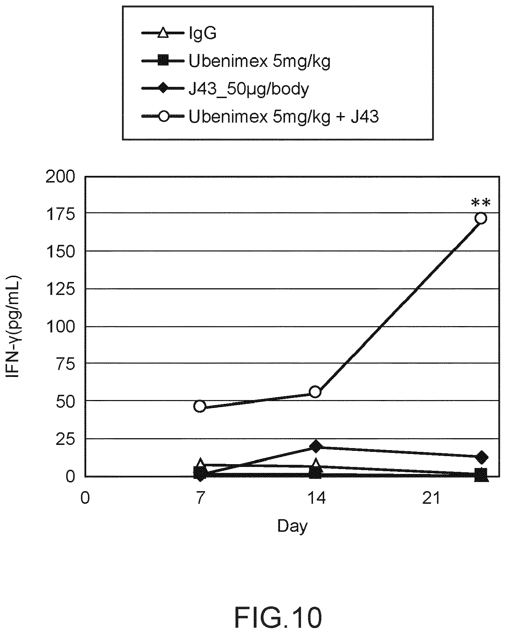

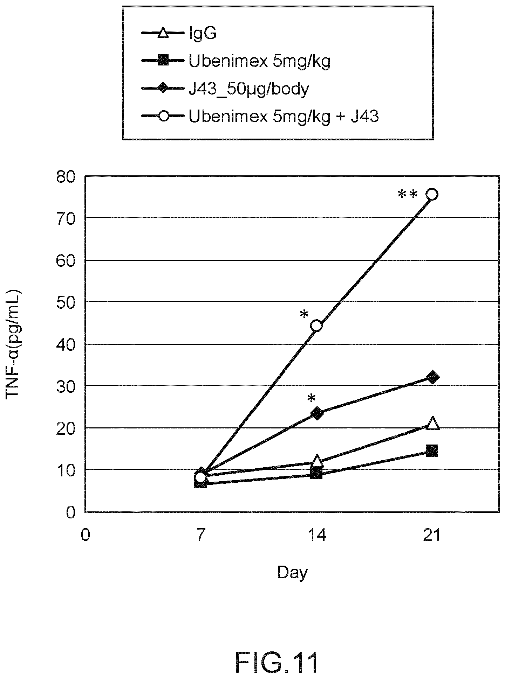

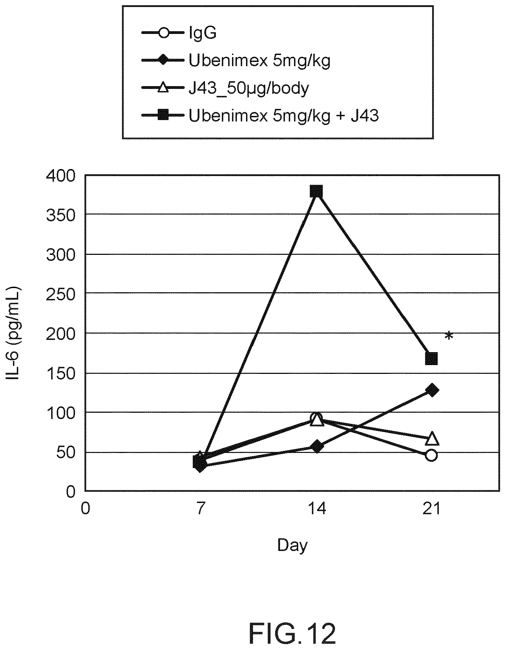

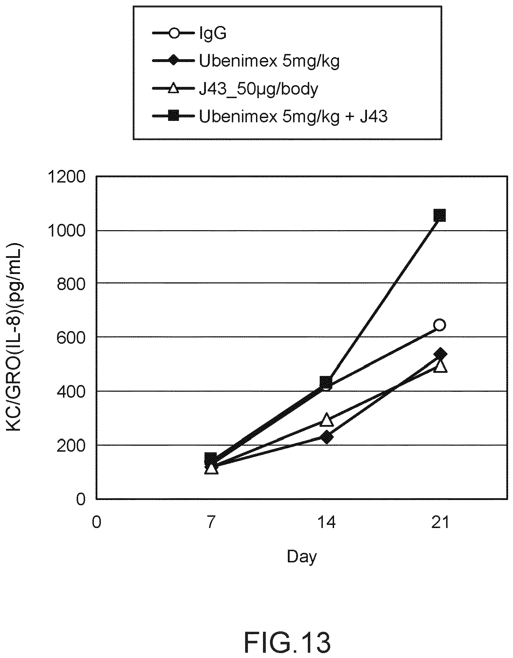

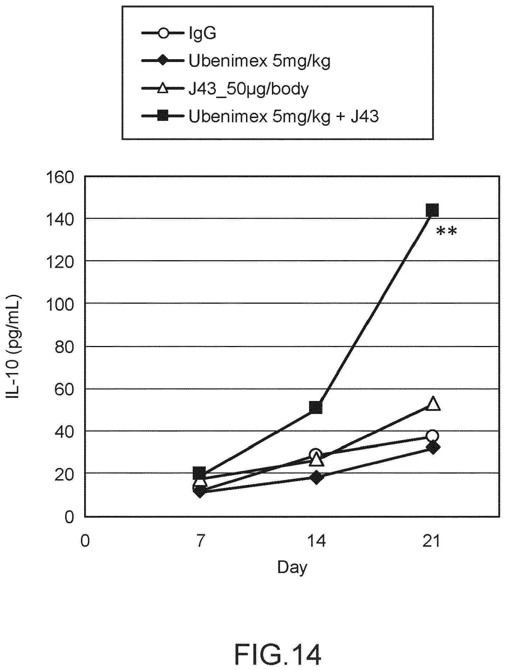

Blood was collected from the jugular vein with a syringe to which a heparin sodium solution (MOCHIDA PHARMACEUTICAL CO., LTD.) was added and a needle 7, 14, 21, or 24 days after the transplantation. The collected blood was centrifuged, and cytokines were measured with the obtained plasma by ELISA or electrochemiluminescence. The IFN-γ concentration was measured with a LBIS Mouse IFN-γ ELISA kit (FUJIFILM Wako Shibayagi Corporation). The concentration of TNF-α, IL-6, KC/GRO (IL-8), and the IL-10 were measured with a V-PLEX Proinflammatory Panel 1 Mouse Kit (MESO SCALE DIAGNOSTICS, LLC.). The transitions thereof were shown in FIG. 10 to FIG. 14.

The control group and the treated groups were subjected to the Wilcoxon tests using the statistical analysis software EXSUS (ver. 10.0, EPS Corporation) for statistical analysis. Two-sided statistical analysis was performed at significance levels of P < 0.05 and P < 0.01.

In the combined use group of ubenimex and anti-PD-1 antibody, the amounts of cytokines produced increased over time. The combined use group exhibited a clear effect of reinforcing the production of IFN-γ, TNF-α, IL-6, KC/GRO (IL-8), and IL-10 as compared with the control group, the treated group of ubenimex alone, and the treated group of the anti-PD-1 antibody alone (FIG. 10 to FIG. 14). With respect to the P values of the combined use group to the control group 21 or 24 days after the transplantation, the P value of IFN-γ was 0.003, the P value of TNF-α was 0.045, the P value of IL-6 was 0.031, and the P value of IL-10 were 0.008.

IFN-γ, TNF-α, IL-6, and IL-8 are known as antitumor cytokines or inflammatory cytokines, which are considered to serially activate the immune system network and contribute to antitumor effect. IL-10 is known as an anti-inflammatory cytokine, which regulates inflammatory response. Since, in Example 3, the combined use group of ubenimex and the anti-PD-1 antibody exhibited a tumor growth inhibition effect that was stronger than the treated group of each single agent, it was believed that the combined administration of ubenimex and the anti-PD-1 antibody brought an increase in the production of IFN-γ, TNF-α, IL-6, and KC/GRO (IL-8), and contributed to tumor growth inhibition. It was conjectured that IL-10 increased in response to the increase in the production of the above-mentioned cytokines compensatorily, and regulated an excessive inflammatory response in the living body.

## Claims

1. An antitumor agent to be administered in combination with an anti-PD-1 antibody, an anti-PD-L1 antibody, and/or an anti-CTLA-4 antibody, which are immune checkpoint inhibitors, comprising:
ubenimex as an active ingredient.

2. The antitumor agent according to claim 1, wherein a dosage of ubenimex is a dosage such that an area under a blood concentration-time curve (AUC) at the time of single administration is 3.00 to 50.00 µg•hr/mL.

3. The antitumor agent according to claim 1 or 2, wherein ubenimex is for daily administration for usage.

4. An antitumor agent to be administered in combination with ubenimex, comprising:
one selected from the group consisting of an anti-PD-1 antibody, an anti-PD-L1 antibody, and an anti-CTLA-4 antibody, which are immune checkpoint inhibitors, as an active ingredient.

5. The antitumor agent according to claim 4, wherein a dosage of ubenimex is a dosage such that an area under a blood concentration-time curve (AUC) at the time of single administration is 3.00 to 50.00 µg•hr/mL.

6. A medicament to be administered in combination with an anti-PD-1 antibody, an anti-PD-L1 antibody, and/or an anti-CTLA-4 antibody, which are immune checkpoint inhibitors, to enhance antitumor cytokine production, comprising:
ubenimex as an active ingredient.

7. The medicament according to claim 6, wherein a dosage of ubenimex is a dosage such that an area under a blood concentration-time curve (AUC) at the time of single administration is 3.00 to 50.00 µg•hr/mL.

8. A medicament to be administered in combination with an anti-PD-1 antibody, an anti-PD-L1 antibody, and/or an anti-CTLA-4 antibody, which are immune checkpoint inhibitors, to increase tumor-infiltrating lymphocytes, comprising:
ubenimex as an active ingredient.

9. The medicament according to claim 8, wherein a dosage of ubenimex is a dosage such that an area under a blood concentration-time curve (AUC) at the time of single administration is 3.00 to 50.00 µg•hr/mL.

10. A medicament comprising:
ubenimex as an active ingredient,
wherein the medicament is administered in combination with an anti-PD-1 antibody, an anti-PD-L1 antibody, and/or an anti-CTLA-4 antibody, which are immune checkpoint inhibitors, to be used for the treatment of a malignant tumor selected from the group consisting of small cell lung cancer, non-small cell lung cancer, esophagus cancer, head and neck cancer, brain tumor, colorectal cancer, colon adenocarcinoma, gastric cancer, breast cancer, hepatocellular cancer, pancreatic cancer, biliary tract cancer, kidney cancer, prostate cancer, bladder cancer, ovarian cancer, cervical cancer, thyroid cancer, and melanoma.

11. The medicament according to claim 10, wherein a dosage of ubenimex is a dosage such that an area under a blood concentration-time curve (AUC) at the time of single administration is 3.00 to 50.00 µg•hr/mL.

12. The medicament according to claim 10 or 11, wherein ubenimex is for daily administration for usage.
